# EUROPEAN PATENT APPLICATION

(11) **EP 4 173 532 A1**
(43) Date of publication of application: **03.05.2023**
(21) Application number: 21828881.9
(22) Date of filing: 20.05.2021
(51) Int. Cl.: A47K 10/02, A41B 9/12, A41B 17/00, A61F 13/15

(54) **MENSTRUAL SANITARY TOWEL**

(30) Priority: 26.06.2020 ES 202031394 U
(71) Applicant: Sanchez Gonzalez, Angela, 29780 Nerja Malaga (ES)
(72) Inventor: Sanchez Gonzalez, Angela, 29780 Nerja Malaga (ES)
(74) Representative: Espiell Gomez, Ignacio
(86) International application number: PCT/ES2021/070363
(87) International publication number: WO 2021/260237

(57) **Abstract**

A menstrual sanitary towel which, applicable for use during the days of menstruation, is worn to absorb possible losses without becoming detached while the user dries herself on leaving the shower or bathtub, manufactured from towelling fabric or similar (2) and made as an item of clothing in the form of panties; at the internal part thereof, at least at the central portion that is in contact with the private parts of the user when she wears it, it incorporates one or more inserts of absorbent fabric (3). Preferably, the absorbent insert fabric (3) is formed by one or more layers of absorbent fabrics. Preferably, the menstrual sanitary towel includes a loop (4) for hanging the same.

## Description

### OBJECT OF THE INVENTION

As expressed in the title of the present specification, the invention relates to a menstrual sanitary towel that provides, to the function for which it is intended, advantages and features which are described in detail below.

The object of the present invention relates to a towel which, specially designed for use during the days that menstruation lasts as a towel for drying off on leaving the shower or bathtub, has the particularity of being in the form of panties that are worn to absorb possible blood losses without becoming detached while the user dries the rest of her body, fixes her hair and/or primps, allowing her to carry out body hygiene in complete safety and peace of mind, avoiding staining of towels, bathroom rugs, the floor or the skin itself after the shower or bath.

### FIELD OF APPLICATION OF THE INVENTION

The field of application of the present invention falls within the sector of the industry dedicated to the manufacture of sanitary hygiene clothes and items of clothing, as well as the industry dedicated to sanitary hygiene for women in general.

### BACKGROUND OF THE INVENTION

As is known, during menstruation, blood losses occur which, if a pad, tampon or other means of sanitary absorption is not used, can cause the staining of any surface or item of clothing with which the woman is in contact. For this reason, on leaving the shower or bathtub, a moment in which such means of absorption are not usually worn, precisely to be able to carry out correct sanitary hygiene, is a moment in which said staining can occur, that is, staining of the towel to be used to dry off, of the bathroom mat, of the floor and of the skin on the legs, depending on the degree of flow that occurs, with the consequent discomfort that this can cause, particularly when travelling.

Therefore, the objective of the present invention is to provide a towel specifically designed to avoid said staining which, in addition, is in the form of panties, allowing it to be held up, covering the private parts and effectively absorbing possible blood loss while the rest of the body is dried, hair is dried and fixed and/or personal grooming that is necessary is performed, with the peace of mind of not staining anything other than the menstrual towel itself, unlike bidet towels or other cloths that are normally used to avoid this problem and that do not represent an optimal or adequate solution.

Moreover, and as a reference to the current state of the art, it should be noted that although different types and models of towels, as well as different types and models of panties are known, at least by the applicant, the existence of a towel presenting the same or similar technical features as those presented by the towel claimed here is unknown.

### DESCRIPTION OF THE INVENTION

The menstrual sanitary towel proposed by the invention is configured as the ideal solution to the aforementioned objective, the characterizing details that make it possible and that distinguish it being suitably set out in the final claims attached to the present description.

Specifically, as noted above, the invention proposes a towel, or similar drying fabric analogous to towelling fabric, which is in the form of panties specially designed for use during the days of menstruation to dry off on leaving the shower or bathtub, providing the advantage of being able to wear it to absorb possible blood losses through the absorbent fabric that it includes at the internal part thereof without becoming detached while the user dries the rest of her body, fixes her hair and /or primps, allowing her to carry out body hygiene on leaving the shower or bathtub in complete safety and peace of mind, avoiding staining of bath towels, bath mats, the floor or the skin itself.

For this purpose and more specifically, the menstrual sanitary towel is a towel manufactured from cotton towelling fabric or similar fabric made in the form of panties; at the internal part thereof, at least in the central portion that is in contact with the private parts of the user when she wears it, it incorporates an absorbent insert fabric, preferably one or more layers of absorbent fabrics such as those used for cloth menstrual pads on the market, whether natural or synthetic.

Therefore, the towel of the invention represents an optimal solution to the problem described, for which, until now, there was no solution.

Its size and shape similar to that of panties make it a very practical item to transport on trips or away-from-home stays, as it is discreet and takes up very little space.

It is a washable towel, which allows reuse and hygiene.

It avoids staining towels, mats or the woman's own skin.

Its manufacture from towelling fabric allows to continue with the drying of the skin that desired after a shower or bath.

This is a solution suited to any menstruating woman.

Preferably, it also includes a practical loop on the side that allows the towel to be hung on a bathroom hook and accessed without having to get out of the shower before putting it on.

In the preferred embodiment, it is 100% breathable.

### DESCRIPTION OF THE DRAWINGS

To complement the description that is being made and for the purpose of aiding to better understand the features of the invention, a drawing is attached to the present specification as an integral part thereof, in which the following is depicted in an illustrative and non-limiting manner:
Sole Figure number 1 shows a schematic front elevational depiction of an exemplary embodiment of the menstrual sanitary towel object of the invention, where its configuration and the main parts it comprises can be seen.

### PREFERRED EMBODIMENT OF THE INVENTION

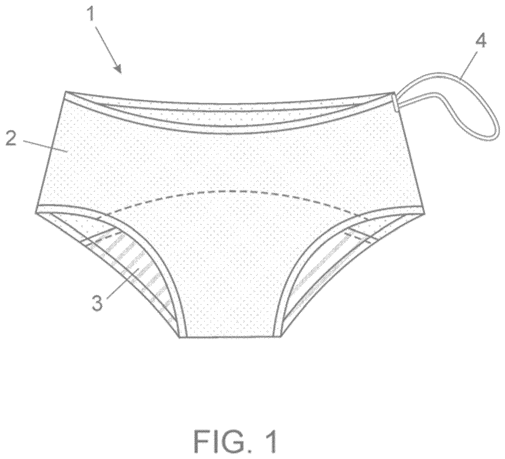

In view of the described Figure 1, and in accordance with the numbering adopted, a non-limiting exemplary embodiment of the menstrual sanitary towel of the invention, which comprises what is described in detail below, can be seen in said figure.

Thus, as can be seen in said figures, the invention is distinguished by consisting of a towel (1) manufactured from towelling fabric (2), preferably cotton towelling fabric, or similar personal drying fabric. It is made as an item of clothing in the form of panties; at the internal part thereof, at least in the central portion that is in contact with the private parts of the user when she wears it, it incorporates an absorbent insert fabric (3), preferably formed by one or more layers of natural absorbent fabrics such as those used for cloth menstrual pads, but without ruling out that they may be synthetic.

In addition, in the preferred embodiment of the invention, the described towel (1) includes a loop (4) for hanging the same.

Preferably, the towel (1) is manufactured from 100% breathable fabric.

Having sufficiently described the nature of the present invention, as well as the way of putting it into practice, it is not considered necessary to further expand on its explanation so that any expert in the field can understand its scope and the advantages derived from it.

## Claims

1. A menstrual sanitary towel which, applicable for use during the days of menstruation, is worn to absorb possible losses without becoming detached while the user dries herself on leaving the shower or bathtub, is **characterized in that** it is manufactured from towelling fabric or similar (2) and made as an item of clothing in the form of panties; at the internal part thereof, at least at the central portion that is in contact with the private parts of the user when she wears it, it incorporates one or more inserts of absorbent fabric ( 3).

2. The menstrual sanitary towel according to claim 1, **characterized in that** the towelling fabric (2) with which it is manufactured is cotton towelling fabric or similar drying fabric.

3. The menstrual sanitary towel according to claim 1 or 2, **characterized in that** the absorbent insert fabric (3) is formed by one or more layers of absorbent fabrics.

4. The menstrual sanitary towel according to any of the preceding claims, **characterized in that** it includes a loop (4) for hanging the same.

5. The menstrual sanitary towel according to any of the preceding claims, **characterized in that** said towel (1) is preferably manufactured from 100% breathable fabric.
